# EUROPEAN PATENT APPLICATION

(11) **EP 2 269 970 A1**
(43) Date of publication of application: **05.01.2011**
(21) Application number: 10010042.9
(22) Date of filing: 18.02.1998
(51) Int. Cl.: C07C 19/08, C07C 19/10, C07C 17/07, C07C 17/383, C07C 41/22, C07C 43/12

(54) **Azeotropic compositions comprising 1,1,1,2,3,3,3-heptafluoropropane and processes using said compositions**

(30) Priority: 19.02.1997 US 38430 P; 16.06.1997 US 49723 P; 25.08.1997 US 56795 P
(62) Divisional of application: 03078647.9
(71) Applicant: E. I. du Pont de Nemours and Company, Wilmington, DE 19898 (US)
(72) Inventor: Rao, V. N. Mallikarjuna, Wilmington, DE 19809 (US); Sievert, Allen, Capron, Elkton, MD 21921 (US)
(74) Representative: Towler, Philip Dean

(57) **Abstract**

Disclosed are compositions and a process for producing compositions comprising (c1), CF₃CHFCF₃, CF₃CH₂CF₃ or CHF₂CH₂CF₃ and (c2) at least one saturated halogenated hydrocarbon and/or ether having the formula:

CₙH_{2n+2-a-b}ClₐF_{b}O_{c}

wherein n is an integer from 1 to 4, a is an integer from 0 to 2n+1, b is an integer from 1 to 2n+2-a, and c is 0 or 1, provided that when c is 1 then n is an integer from 2 to 4, and provided that component (c2) does not include the selected component (c1) compound, wherein the molar ratio of component (c2) to component (c1) is between about 1:99 and a molar ratio of HF to component (c1) in an azeotrope or azeotrope-like composition of component (c1) with HF. The process involves (A) combining (i) the azeotropic composition with (ii) the fluorination precursor to component (c2); and (B) reacting a sufficient amount of the HF from the azeotrope or azeotrope-like composition (i) with precursor component (ii) to provide a composition containing components (c1) and (c2) in the desired ratio. The compositions include at least two (c1) compounds, at least one of which is an ether.

## Description

### FIELD OF THE INVENTION

This invention relates to fluorine-substituted hydrocarbons, and more particularly to processes for producing CF₃CH=CF₂, CF₃CCl=CF₂ and saturated derivatives thereof such as CF₃CH₂CF₃, CF₃CH₂CHF₂ and CF₃CHFCF₃, and to compositions comprising the saturated derivatives (e.g., azeotropes of said saturated derivatives with HF and uses of said azeotropes).

### BACKGROUND

A number of chlorine-containing halocarbons are considered to be detrimental toward the Earth's ozone layer. There is a world-wide effort to develop materials having lower ozone depletion potential that can serve as effective replacements. For example, the hydrofluorocarbon, 1,1,1,2-tetrafluoroethane (HFC-134a) is being used as a replacement for dichlorodifluoromethane (CFC-12) in refrigeration systems. The production of hydrofluorocarbons (i.e., compounds containing only carbon, hydrogen and fluorine), has been the subject of considerable interest to provide environmentally desirable products for use as solvents, blowing agents, refrigerants, cleaning agents, aerosol propellants, heat transfer media, dielectrics, fire extinguishants and power cycle working fluids (see, e.g., PCT International Publication No. WO 93/02150).

### SUMMARY OF THE INVENTION

A process is provided in accordance with this invention for producing pentafluoropropenes of the formula CF₃CX=CF₂, where X is H or Cl. The process comprises hydrodehalogenating CF₃CCl₂CF₃ with hydrogen at an elevated temperature in the vapor phase over a catalyst comprising at least one component selected from the group consisting of elemental metals, metal oxides, metal halides and metal oxyhalides; wherein the metal of said hydrodehalogenation catalyst component is selected from copper, nickel, chromium and mixtures thereof and the halogen of said halides and said oxyhalides is selected from fluorine, chlorine and mixtures thereof.

This invention further provides a process for producing the hydrofluorocarbon CF₃CHFCF₃. This process comprises (a) hydrodehalogenating CF₃CCl₂CF₃ with hydrogen as indicated above to produce a product comprising CF₃CCl=CF₂, CF₃CH=CF₂, HCl and HF; and (b) reacting the CF₃CCl=CF₂ produced in (a) with HF to produce CF₃CHFCF₃.

This invention further provides a process for producing the hydrofluorocarbon CF₃CH₂CHF₂. This process comprises (a) hydrodehalogenating CF₃CCl₂CF₃ with hydrogen as indicated above to produce a product comprising CF₃CCl=CF₂, CF₃CH=CF₂, HCl and HF; and (b) reacting at least one of said CF₃CCl=CF₂ and CF₃CH=CF₂ produced in (a) in the vapor phase with hydrogen to produce CF₃CH₂CHF₂.

This invention further provides a process for producing CF₃CH₂CF₃. This process comprises (a) hydrodehalogenating CF₃CCl₂CF₃ with hydrogen as indicated above to produce a product comprising CF₃CCl=CF₂, CF₃CH=CF₂, HCl and HF; and (b) reacting the CF₃CH=CF₂ produced in (a) with HF to produce CF₃CH₂CF₃.

Azeotropic compositions (e.g., an azeotropic composition consisting essentially of from about 29.9 to about 41.3 mole percent HF and from about 70.1 to 58.7 mole percent CF₃CHFCF₃) are also provided which comprise CF₃CHFCF₃ and HF, wherein said HF is present in an amount effective to form an azeotropic combination with said CF₃CHFCF₃.

The present invention further provides a process for recovering HF from a product mixture comprising HF and CF₃CHFCF₃. The process comprises (1) distilling the product mixture to remove all products which have a lower boiling point than the lowest boiling azeotrope containing HF and CF₃CHFCF₃; and (2) distilling said azeotrope to recover HF as an azeotropic composition containing HF and CF₃CHFCF₃.

This invention further provides a process for producing compositions comprising (c1) a compound selected from the group consisting of CF₃CHFCF₃, CF₃CH₂CF₃ and CHF₂CH₂CF₃ and (c2) at least one saturated compound selected from halogenated hydrocarbons and ethers having the formula:

CₙH_{2n+2-a-b}ClₐF_{b}O_{c}

wherein n is an integer from 1 to 4, a is an integer from 0 to 2n+1, b is an integer from 1 to 2n+2-a, and c is 0 or 1, provided that when c is 1 then n is an integer from 2 to 4, and provided that component (c2) does not include the selected component (c1) compound, wherein the molar ratio of component (c2) to component (c1) is between about 1:99 and a molar ratio of HF to component (c1) in an azeotrope or azeotrope-like composition of component (c1) with HF. This process comprises (A) combining (i) said azeotrope or azeotrope-like composition with (ii) at least one fluorination precursor compound, wherein the precursor component (ii) is the fluorination precursor to component (c2); and (B) reacting a sufficient amount of the HF from the azeotrope or azeotrope-like composition (i) with precursor component (ii) to provide a composition containing components (c1) and (c2) in said ratio.

In addition, compositions are provided comprising: (c1) a compound selected from the group consisting of CF₃CHFCF₃, CF₃CH₂CF₃ and CHF₂CH₂CF₃; and (c2) at least two saturated compounds selected from halogenated hydrocarbons and ethers having the formula:

CₙH_{2n+2-a-b}ClₐF_{b}O_{c}

wherein n is an integer from 1 to 4, a is an integer from 0 to 2n+1, b is an integer from 1 to 2n+2-a, and c is 0 or 1, provided that when c is 1 then n is an integer from 2 to 4, provided that component (c2) does not include the selected component (c1) compound and provided that c is 1 for at least one of the component (c2) compounds, wherein the molar ratio of component (c2) to component (c1) is between 1:99 and 41.3:58.7 when component (c1) is CF₃CHFCF₃, between 1:99 and 59:41 when component (c1) is CF₃CH₂CF₃, and between 1:99 and 84:16 when component (c1) is CHF₂CH₂CF₃.

### DETAILED DESCRIPTION

This invention provides a process for producing 1,1,1,3,3-pentafluoropropane (i.e., CF₃CH₂CHF₂ or HFC-245fa) using 2,2-dichloro-1,1,1,3,3,3-hexafluoropropane (i.e., CF₃CCl₂CF₃ or CFC-216aa).

The present invention includes the hydrodehalogenation of CFC-216aa in a manner which removes a single fluorine from an end carbon while removing at least one chlorine from the internal carbon to produce CF₃CCl=CF₂ (CFC-1215xc) and CF₃CH=CF₂ (HCFC-1225zc). This hydrodehalogenation generally produces a product comprising CF₃CCl=CF₂, CF₃CH=CF₂, HF and HCl, and involves the use of advantageously catalytic components employing copper, nickel and/or chromium. Suitable components include halides such as CuF, CuCl, CuCl₂, CuClF, NiF₂, NiCl₂, NiClF, CrF₃, CrCl₃, CrCl₂F and CrClF₂; oxides such as CuO, NiO, and Cr₂O₃; and oxyhalides such as copper oxyfluoride and chromium oxyfluoride. Oxyhalides may be produced by conventional procedures such as, for example, halogenation of metal oxides.

The catalysts of this invention may contain other components, some of which are considered to improve the activity and/or longevity of the catalyst composition. Preferred catalysts include catalysts which are promoted with compounds of molybdenum, vanadium, tungsten, silver, iron, potassium, cesium, rubidium, barium or combinations thereof. Also of note are chromium-containing catalysts which further contain zinc and/or aluminum or which comprise copper chromite.

The catalyst may be supported or unsupported. Supports such as metal fluorides, alumina and titania may be advantageously used. Particularly preferred are supports of fluorides of metals of Group IIB, especially calcium. A preferred catalyst consists essentially of copper, nickel and chromium oxides (each of said oxides being preferably present in equimolar quantities) preferably promoted with potassium salt, on calcium fluoride.

An especially preferred catalyst contains proportionally about 1.0 mole CuO, about 0.2 to 1.0 mole NiO, about 1 to 1.2 moles Cr₂O₃ on about 1.3 to 2.7 moles CaF₂, promoted with about 1 to 20 weight%, based on the total catalyst weight, of an alkali metal selected from K, Cs, and Rb (preferably K). When K is the promoter, the preferred amount is from about 2 to 15 weight% of the total catalyst.

This catalyst can be prepared by coprecipitating, from an aqueous medium, salts of copper, nickel and chromium (and optionally aluminum and zinc), with and preferably on calcium fluoride; washing, heating and drying the precipitate. An alkali metal compound (e.g., KOH, KF or K₂CO₃) is then deposited on the dried precipitate, followed by calcination to convert the copper, nickel and chromium to the respective oxides. Any soluble copper, nickel and chromium compound may be used, but the chlorides and nitrates are preferred, with the nitrates being especially preferred. Alternatively, promoters such as KOH, KF and K₂CO₃ may be added prior to co-precipitation.

Another group of catalysts which may be used for the conversion of CF₃CCl₂CF₃ contains proportionally about 1.0 mole CuO, about 0.2 to 1.0 mole NiO, about 1 to 1.2 moles Cr₂O₃, about 0.4 to 1.0 mole MoO₃, and about 0.8 to 4.0 mole CaF₂, optionally promoted with at least one compound from the group consisting of MgF₂, MnF₂, and BaF₂. Palladium or WO₃ may also be present.

The catalyst may be granulated, pressed into pellets, or shaped into other desirable forms. The catalyst may contain additives such as binders and lubricants to help insure the physical integrity of the catalyst during granulating or shaping the catalyst into the desired form. Suitable additives include carbon and graphite. When binders and/or lubricants are added to the catalyst, they normally comprise about 0.1 to 5 weight percent of the weight of the catalyst.

The catalyst may be activated prior to use by treatment with hydrogen, air, or oxygen at elevated temperatures. After use for a period of time in the process of this invention, the activity of the catalyst may decrease. When this occurs, the catalyst may be reactivated by treating it with hydrogen, air or oxygen, at elevated temperature in the absence of organic materials.

The molar ratio of hydrogen to CF₃CCl₂CF₃ fed to the process typically ranges from about 1:1 to about 30:1, and is preferably at least about 3:1.

The hydrodehalogenation process of CF₃CCl₂CF₃ is suitably conducted at a temperature in the range of from about 300°C to 450°C, preferably from about 350°C to about 400°C. The contact time of reactants with the catalyst bed (i.e., the volume of the catalyst bed divided by the volumetric flow rate at the temperature and pressure of the reaction) is typically from about 5 seconds to about 4 minutes.

The product from the hydrodehalogenation reaction of CF₃CCl₂CF₃ comprises CF₃CH=CF₂, CF₃CCl=CF₂, HCl and HF and typically other compounds such as unreacted CF₃CCl₂CF₃ and partially reacted compounds such as CF₃CHClCF₃. These products may be separated by conventional means such as distillation and/or decantation and the components may be used individually. For example, CF₃CH=CF₂ (HFC-1225zc) may be used as a co-monomer for producing fluorine-containing polymers. Unreacted starting material (CFC-216aa) can be recycled to the hydrodehalogenation reactor.

Products from the hydrodehalogenation reaction of CF₃CCl₂CF₃ which contain the unreacted CFC-216aa and optionally also contain the partially reacted compound CF₃CHClCF₃, after isolation from the unsaturated hydrodehalogenation products, can also be further reacted with hydrogen (e.g., using a conventional supported palladium hydrogenation catalyst). This hydrogenation can be used to produce CF₃CH₂CF₃ (HFC-236fa), a useful fire extinguishant. If desired, the amount of HFC-236fa produced in this manner can be increased by decreasing the contact time of the CFC-216aa reactant in the hydrodehalogenation reactor.

CF₃CCl=CF₂ (CFC-1215xc) may be reacted with HF to produce CF₃CHFCF₃ (HFC-227ea), which is a useful fire extinguishant. The reaction is typically conducted at an elevated temperature in either the liquid or vapor phase using a fluorination catalyst. For example, CF₃CCl=CF₂ may be reacted with HF in the liquid phase at a temperature of from about 100 to 175°C over a pentavalent antimony catalyst (e.g., SbF₅) to produce CF₃CHFCF₃; or CF₃CCl=CF₂ may be reacted with HF in the vapor phase at a temperature of from about 300 to 400°C over an unsupported or supported trivalent chromium catalyst (e.g., Cr₂O₃ or Cr₂O₃/AlF₃). Of note are embodiments where the HF produced from the hydrodehalogenation of CF₃CCl₂CF₃ is used for CF₃CHFCF₃ production. Preferably, however, the mole ratio of HF to CF₃CCl=CF₂ used for CF₃CHFCF₃ production is at least about 5:1.

The reaction products from the hydrofluorination may be separated by conventional techniques, such as distillation. CF₃CHFCF₃ may form azeotropic combinations with HF and/or HCl; and conventional decantation/distillation may be employed if further purification of CF₃CHFCF₃ is desired.

An azeotrope is a liquid mixture that exhibits a maximum or minimum boiling point relative to the boiling points of surrounding mixture compositions. A characteristic of minimum boiling azeotropes is that the bulk liquid composition is the same as the vapor compositions in equilibrium therewith, and distillation is ineffective as a separation technique. It has been found, for example, that CF₃CHFCF₃ (HFC-227ea) and HF form a minimum boiling azeotrope. This azeotrope can be produced as a co-product with HFC-227ea. As discussed further below, compositions may be formed which consist essentially of azeotropic combinations of hydrogen fluoride with HFC-227ea. These include a composition consisting essentially of from about 29.9 to 41.3 mole percent HF and from about 70.1 to 58.7 mole percent HFC-227ea (which forms an azeotrope boiling at a temperature between about -25°C and about 100°C at a pressure between about 77 kPa and about 3764 kPa). The hydrofluorocarbons (e.g., HFC-227ea) can be separated from the HF in such azeotropes by conventional means such as neutralization and decantation. However, azeotropic compositions of hydrofluorocarbons and HF (e.g., an azeotrope recovered by distillation of fluorination reactor effluent) are useful as recycle to the fluorination reactor, where the recycled HF can function as a reactant and the recycled hydrofluorocarbon can function to moderate the temperature effect of the heat of reaction. Thus, for example, the process of this invention for producing CF₃CHFCF₃ can further comprise the steps of recovering a portion of the CF₃CHFCF₃ as an azeotropic composition of CF₃CHFCF₃ and HF and recycling said azeotropic composition to the reactor.

CF₃CH=CF₂ (CFC-1225zc) may be reacted with HF to produce CF₃CH₂CF₃ (HFC-236fa), which is a useful fire extinguishant. The reaction is typically done in either the liquid or vapor phase with or without using a fluorinating catalyst. For example, CF₃CH=CF₂ may be reacted with HF in the liquid phase at a temperature of from about 20°C to about 175°C in the absence of a catalyst to produce CF₃CH₂CF₃; or CF₃CH=CF₂ may be reacted with HF in the liquid phase at a temperature of from about 0°C to about 175°C over a polyvalent metal halide catalyst (e.g., SbF₅, AlF₃, MoF₅, TaF₅, NbF₅, SnCl₄, SbCl₅ or TiCl₄) to produce CF₃CH₂CF₃. Also, CF₃CH=CF₂ may be reacted with HF in the vapor phase at a temperature of from about 150°C to about 400°C in the absence of a catalyst to produce CF₃CH₂CF₃; or CF₃CH=CF₂ may be reacted with HF in the vapor phase at a temperature of from about 50°C to about 400°C over an unsupported or supported trivalent chromium catalyst (e.g., Cr₂O₃ or Cr₂O₃/AlF₃) or other vapor phase fluorination catalysts such as AlF₃, carbon, or a transition metal (e.g., Co, Mn, and/or Cr) supported on AlF₃. Of note are embodiments where the HF produced from the hydrodehalogenation of CF₃CCl₂CF₃ is used for CF₃CH₂CF₃ production. Preferably, however, the mole ratio of HF to CF₃CH=CF₂ used for CF₃CH₂CF₃ production is at least about 1:1. Reference is made to U.S. Patent No. 5,563,304 for a discussion of vapor phase hydrofluorination.

The reaction products from the hydrofluorination may be separated by conventional techniques, such as distillation. CF₃CH₂CF₃ forms an azeotrope with HF (see U.S. Patent No. 5,563,304) and may also form an azeotrope with HCl; and conventional decantation/distillation may be employed if further purification of CF₃CH₂CF₃ is desired. The azeotropic compositions of CF₃CH₂CF₃ include a composition consisting essentially of from about 59 to 37 mole percent HF and from about 41 to 63 mole percent CF₃CH₂CF₃ (which forms an azetrope having a boiling point from about -25°C at 44 kPa to about 100°C at 2900 kPa).

In another embodiment, the HFC-1225zc and/or CFC-1215xc produced by the hydrodehalogenation reaction of this invention may be reacted with hydrogen in the vapor phase to produce CF₃CH₂CHF₂. The reaction of CF₃CCl=CF₂ and/or CF₃CH=CF₂ with hydrogen can employ a hydrogenation catalyst. Suitable hydrogenation catalysts include those which contain a metal (e.g., a Group VIII metal or rhenium). The metal may be supported (e.g., Pd supported on alumina, aluminum fluoride, or carbon) or may be unsupported (e.g., Raney nickel). Carbon-supported metal catalysts are preferred, with Pd/C being particularly preferred. The carbon support is preferably washed with acid prior to depositing the metal on it. Procedures for preparing a catalyst of Group VIII metal or rhenium on an acid-washed carbon support are disclosed in U.S. Patent No. 5,136,113, the entire contents of which are hereby incorporated by reference.

Of note is a process where CF₃CCl=CF₂ and/or CF₃CH=CF₂ is contacted with hydrogen in the presence of a hydrogenation catalyst and in the presence of HCl and HF. The CF₃CH=CF₂ and/or CF₃CCl=CF₂ may be isolated from the hydrodehalogenation reaction effluent by distillation if desired, and then passed to the hydrogenation step, with HCl and HF being separately added in the hydrogenation step. However, it is preferred to pass the HCl and HF from the hydrodehalogenation, and more preferably the entire effluent from the hydrodehalogenation of CF₃CCl₂CF₃ (including the CF₃CCl=CF₂, CF₃CH=CF₂, HCl and HF), with hydrogen over the hydrogenation catalyst. While the hydrogenation reaction proceeds even in the absence of HCl and HF, the HCl and HF present during the hydrogenation step moderates the hydrogenation reaction. In any case, in accordance with this invention, CF₃CH₂CHF₂ may be produced from CF₃CCl₂CF₃ without separation and removal of HCl and HF prior to CF₃CH₂CHF₂ production. In addition, passing the entire effluent from the hydrodehalogenation step on to the hydrogenation step avoids handling concerns associated with olefinic halogenated compounds as well as HCl and HF. The HCl and HF of the hydrogenation effluent is available for use along with other compounds thereof. For example, the HF is available for azeotropic combination with the fluorinated hydrocarbon compounds of the effluent from the hydrogenation reaction.

The contact of said hydrodehalogenation effluent with hydrogen in the presence of a hydrogenation catalyst and HCl and HF is suitably conducted at a temperature in the range of from about 50°C to about 300°C, and preferably from about 50°C to about 200°C. Contact time is typically from about 5 to 100 seconds, preferably about 10 to 30 seconds.

The molar ratio of hydrogen to CF₃CH=CF₂ in the hydrodehalogenation effluent typically is in the range from about 1:1 to about 50:1, and is preferably from about 1.5:1 to about 25:1, and more preferably from about 2:1 to about 10:1. Normally, at least about 100 ppm each of HCl and HF is present; and typically for each mole of CF₃CH=CF₂, the hydrodehalogenation effluent also contains two moles of HCl and one mole of HF, especially when the entire effluent from the hydrodehalogenation step is passed to the hydrogenation step.

Hydrogen can be fed to the hydrodehalogenation and/or the hydrogenation steps either in the pure state or diluted with inert gas (e.g., nitrogen, helium or argon).

Alternatively, CF₃CH₂CHF₂ may be produced by reacting the CF₃CH=CF₂ and/or CF₃CCl=CF₂ hydrodehalogenation reaction product with hydrogen in an empty reaction vessel of nickel, iron or their alloys in accordance with the disclosure of U.S. Patent No. 5,364,992, which is incorporated herein in its entirety by reference.

The reaction products from the hydrogenation may be separated by conventional techniques, such as distillation. CF₃CH₂CHF₂ forms an azeotrope with HF (see PCT International Publication No. WO97/05089) and may also form an azeotrope with HCl; and conventional decantation/distillation may be employed if further purification of CF₃CH₂CHF₂ is desired. The azeotropic compositions of CF₃CH₂CHF₂ include a composition consisting essentially of from about 84 to 44 mole percent HF and from about 16 to 56 mole percent CF₃CH₂CHF₂ (which forms an azeotrope having a boiling point from about -50°C at 5.5 kPa to about 130°C at 3853 kPa).

Pressure is not critical for the hydrofluorination, hydrogenation, and hydrodehalogenation processes described above. Atmospheric and superatmospheric pressures (e.g., pressure from about 100 kPa to 7000 kPa) are the most convenient and are therefore preferred.

The hydrofluorination, hydrogenation and hydrodehalogenation reactions may be conducted in any suitable reactor. The reaction vessel should be constructed from materials which are resistant to the corrosive effects of hydrogen fluoride such as Inconel™ nickel alloy and Hastelloy™ nickel alloy.

The CF₃CCl₂CF₃ used as a reactant in this process may be produced by known art methods such as disclosed in U.S. Patent No. 5,057,634.

CF₃CH₂CHF₂ has numerous uses including applications in compositions used as refrigerants, blowing agents, propellants, cleaning agents, and heat transfer agents.

### HFC-227ea/HF Azeotrope

As noted above, the present invention provides a composition which consists essentially of hydrogen fluoride and an effective amount of CF₃CHFCF₃ to form an azeotropic composition with hydrogen fluoride. By effective amount is meant an amount which, when combined with HF, results in the formation of an azeotrope or azeotrope-like mixture. As recognized in the art, an azeotrope or an azeotrope-like composition is an admixture of two or more different components which, when in liquid form under given pressure, will boil at a substantially constant temperature, which temperature may be higher or lower than the boiling temperatures of the components, and which will provide a vapor composition essentially identical to the liquid composition undergoing boiling.

For the purpose of this discussion, azeotrope-like compositions means a composition that behaves like an azeotrope (i.e., has constant-boiling characteristics or a tendency not to fractionate upon boiling or evaporation). Thus, the composition of the vapor formed during boiling or evaporation is the same as or substantially the same as the original liquid composition. Hence, during boiling or evaporation, the liquid composition, if it changes at all, changes only to a minimal or negligible extent. This is to be contrasted with non-azeotrope-like compositions in which during boiling or evaporation, the liquid composition changes to a substantial degree.

Accordingly, the essential features of an azeotrope or an azeotrope-like composition are that at a given pressure, the boiling point of the liquid composition is fixed and that the composition of the vapor above the boiling composition is essentially that of the boiling liquid composition (i.e., no fractionation of the components of the liquid composition takes place). It is also recognized in the art that both the boiling point and the weight percentages of each component of the azeotropic composition may change when the azeotrope or azeotrope-like liquid composition is subjected to boiling at different pressures. Thus, an azeotrope or an azeotrope-like composition may be defined in terms of the unique relationship that exists among components or in terms of the compositional ranges of the components or in terms of exact weight percentages of each component of the composition characterized by a fixed boiling point at a specified pressure. It is also recognized in the art that various azeotropic compositions (including their boiling points at particular pressures) may be calculated (see, e.g., W. Schotte Ind. Eng. Chem. Process Des. Dev. (1980) 19, 432-439). Experimental identification of azeotropic compositions involving the same components may be used to confirm the accuracy of such calculations and/or to modify the calculations at the same or other temperatures and pressures.

It has been found that azeotropes of HFC-227ea and HF are formed at a variety of temperatures and pressures. Between 78 kPa (at a temperature of -25°C) and 3764 kPa (at a temperature of 100°C) azeotropic compositions consisting essentially of HFC-227ea and HF range from about 29.9 mole percent HF (and 70.1 mole percent HFC-227ea) to about 41.3 mole percent HF (and 58.7 mole percent HFC-227ea). An azeotrope of HF and CF₃CHFCF₃ has been found at -10°C and 21.9 psia (151 kPa) consisting essentially of about 40.9 mole percent HF and about 59.1 mole percent HFC-227ea). An azeotrope of HF and CF₃CHFCF₃ has also been found at 70°C and 261.2 psia (1800 kPa) consisting essentially of about 37.0 mole percent HF and about 63.0 mole percent HFC-227ea. Based upon the above findings, it has been calculated that an azeotropic composition of about 41.3 mole percent HF and 58.7 mole percent HFC-227ea can be formed at -25°C and 78 kPa and an azeotropic composition of about 29.9 mole percent HF and 70.1 mole percent HFC-227ea can be formed at 125°C and 3764 kPa. Accordingly, the present invention provides an azeotrope or azeotrope-like composition consisting essentially of from about 29.9 to about 41.3 mole percent HF and from about 70.1 to 58.7 mole percent HFC-227ea, said composition having a boiling point from about -25°C to 78 kPa to about 100°C at 3764 kPa.

Processes may employ azeotropic distillation of HF with a compound selected from the group consisting of CF₃CHFCF₃, CF₃CH₂CF₃ and CHF₂CH₂CF₃. Product mixtures obtained from a variety of sources can be distilled. These sources include product mixtures produced by fluorination with HF of CF₃CF=CF₂ to afford HFC-227ea/HF, fluorination with HF of CCl₃CH₂CCl₃ to afford HFC-236fa/HF and fluorination with HF of CHCl₂CH₂CCl₃ to afford HFC-245fa/HF. The described catalytic fluorination with HF reactions can be done in either the liquid or vapor phase using procedures known in the art. The product mixture may be distilled to remove all products which have a lower boiling point than the lowest boiling azeotrope containing HF and a compound selected from the group consisting of HFC-227ea, HFC-236fa and HFC-245fa. Such low-boiling materials can include, for example, HCl. For continuous processes, distillate and azeotropes with higher boiling points can be advantageously removed from appropriate sections of the distillation column. The lowest boiling azeotrope containing HF and one of the following compounds, CF₃CHFCF₃, CF₃CH₂CF₃ or CHF₂CH₂CF₃ may then be distilled such that HF is recovered as an azeotropic composition containing HF together with one of the following compounds, CF₃CHFCF₃, CF₃CH₂CF₃ or CHF₂CH₂CF₃.

Where the mixture (after distilling components boiling at lower temperatures than the lowest boiling azeotrope of HF with CF₃CHFCF₃) consists essentially of HF and CF₃CHFCF₃, HF may be recovered as an azeotrope consisting essentially of CF₃CHFCF₃ and HF. If excess amounts of CF₃CHFCF₃ or HF remain after azeotropes are recovered from these mixtures, such excess may be recovered as a relatively pure compound. The distillation of azeotropes containing HF and CF₃CHFCF₃ may be done at a wide variety of temperatures and pressures. Typically the temperature is between about -25°C and about 125°C and the pressure is between 78 kPa and 3764 kPa. The process of this invention includes embodiments where azeotropic compositions containing from about 58.7 to about 70.1 mole percent CF₃CHFCF₃ are recovered. HF may be recovered for example, from a product mixture including CF₃CHFCF₃ formed by the reaction of CF₃CF=CF₂ with HF.

Where the mixture (after distilling components boiling at lower temperatures than the lowest boiling azeotrope of HF with CF₃CH₂CF₃) consists essentially of HF and CF₃CH₂CF₃, HF may be recovered as an azeotrope consisting essentially of CF₃CH₂CF₃ and HF. If excess amounts of CF₃CH₂CF₃ or HF remain after azeotropes are recovered from these mixtures, such excess may be recovered as a relatively pure compound. The distillation of azeotropes containing HF and CF₃CH₂CF₃ may be done at a wide variety of temperatures and pressures. Typically the temperature is between about -25°C and about 100°C and the pressure is between 44 kPa and 2900 kPa. The process of this invention includes embodiments where azeotropic compositions containing from about 31 to about 63 mole percent CF₃CH₂CF₃ are recovered. HF may be recovered for example, from a product mixture including CF₃CH₂CF₃ formed by the reaction of CCl₃CH₂CCl₃ with HF.

Where the mixture (after distilling components boiling at lower temperatures than the lowest boiling azeotrope of HF with CHF₂CH₂CF₃) consists essentially of HF and CHF₂CH₂CF₃, HF may be recovered as an azeotrope consisting essentially of CHF₂CH₂CF₃ and HF. If excess amounts of CHF₂CH₂CF₃ or HF remain after azeotropes are recovered from these mixtures, such excess may be recovered as a relatively pure compound. The distillation of azeotropes containing HF and CHF₂CH₂CF₃ may be done at a wide variety of temperatures and pressures. Typically the temperature is between about -50°C and about 130°C and the pressure is between 5.5 kPa and 3850 kPa. The process of this invention includes embodiments where azeotropic compositions containing from about 16 to about 56 mole percent CHF₂CH₂CF₃ are recovered. HF may be recovered for example, from a product mixture including CHF₂CH₂CF₃ formed by the reaction of CHCl₂CH₂CCl₃ with HF.

The HFC-227ea/HF azeotrope, as well as the HFC-236fa/HF and HFC-245fa/HF azeotropes can be used as an HF source to fluorinate numerous compounds. Optionally, such fluorinations can employ a fluorination catalyst. The fluorinations can be done in the liquid phase using typical catalysts such as SbCl₅. The fluorinations can also be done in the vapor phase using typical catalysts such as Cr₂O₃. The following compounds, either individually or in mixed blends, can be fluorinated with the HF azeotrope to provide a variety of compositions wherein the ratio of the fluorination product(s) to a compound selected from the group consisting of CF₃CHFCF₃, CF₃CH₂CF₃ and CHF₂CH₂CF₃ is about 1:99, or more (depending upon the azeotropic combination of CF₃CHFCF₃, CF₃CH₂CF₃ or CHF₂CH₂CF₃ and HF used, and the degree of fluorination).

By fluorination precursors to the component (c2) compound(s) is meant compounds which react with HF (optionally in the presence of a fluorination catalyst) to produce the corresponding component (c2) compound(s). Fluorination precursors include saturated compounds having the formula

CₙH_{2n+2-a-b}Clₐ₊ₓF_{b-x}O_{c}

wherein x is an integer from 1 to b. Examples of saturated precursors and corresponding products are as follows:

| SATURATED PRECURSOR | PRODUCT |
|---|---|
| CH₂Cl₂ | CH₂CF₂ |
| CHCl₂CHCl₂ | CHF₂CHF₂ |
| CF₃CH₂Cl | CF₃CH₂F |
| CH₂ClCF₂CHF₂ | CH₂FCF₂CHF₂ |
| CH₃CF₂CCl₃ | CH₃CF₂CF₃ |
| CHCl₂CH₂CCl₃ | CHF₂CH₂CF₃ |
| CHCl₂OCF₂CHF₂ | CHF₂OCF₂CHF₂ |
| CF₃CHClOCHF₂ | CF₃CHFOCHF₂ |
| CHF₂OCHCl₂ | CHF₂OCHF₂ |
| CClF₂OCHF₂ | CF₃OCHF₂ |

Fluorination precursors also include unsaturated compounds having the formula

CₙH_{2n+1-a-b}Cl_{a+y}F_{b-y-1}O_{c}

wherein y is an integer from 0 to b-1. Examples of unsaturated precursors and corresponding products are as follows:

| UNSATURATED PRECURSOR | PRODUCT |
|---|---|
| CH₂=CF₂ | CH₃CF₃ |
| CH₂=CH₂ | CH₃CH₂F |
| CH₂=CCl₂ | CH₃CCl₂F |
| CF₃CH=CH₂ | CF₃CH₂CH₂F |
| CF₃CCl=CCl₂ | CF₃CHClCClF₂ |
| CF₃CF=CHF | CF₃CHFCHF₂ |
| CF₃CH=CF₂ | CF₃CH₂CF₃ |
| CF₃OCF=CF₂ | CF₃OCHFCF₃ |

Of particular note are processes where for component (b) a is 0 and b is 2n+1, or less.

These fluorinations include processes for producing compositions wherein the molar ratio of component (c2) to CF₃CHFCF₃ is between about 1:99 and about 41.3:58.7; the molar ratio of component (c2) to CF₃CH₂CF₃ is between about 1:99 and about 59:41; and the molar ratio of component (c2) to CHF₂CH₂CF₃ is between about 1:99 and about 84:16. This process comprises (A) combining (i) an azeotrope or azeotrope-like composition consisting essentially of CF₃CHFCF₃, CF₃CH₂CF₃ or CHF₂CH₂CF₃ and HF wherein the ratio of HF to the (c1) component is at least equal to the desired ratio of component (c2) to the respective component (c1) compound with the precursor component (ii).

Of note are embodiments of this fluorination where the CF₃CHFCF₃/HF, CF₃CH₂CF₃/HF or CHF₂CH₂CF₃/HF azeotropes combined with the precursor(s) is obtained by (1) distilling a product mixture comprising HF and a compound selected from the group consisting of CF₃CHFCF₃, CF₃CH₂CF₃ and CHF₂CH₂CF₃ to remove all products which have a lower boiling point than the lowest boiling azeotrope containing HF and said compound; and (2) distilling said azeotrope to recover HF as an azeotropic composition containing HF and said compound. Also of note are processes where the fluorination precursors include precursors for at least two saturated compounds of the formula CₙH₂ₙ₊₂₋ₐ₋-_{b}ClₐF_{b}O_{c}, where c is 1 for at least one of said saturated compounds.

The fluorination product components containing component (c1) and component (c2) may be separated by conventional means such as distillation, selective sorption and/or decantation. The compositions of this invention comprising components (c1) and (c2) (including at least one ether) are useful, for example, as aerosol propellants, fire extinguishants, and/or refrigerants. Some of the compounds of the component (c1)/component (c2) combinations may form HCl azeotropes. The HCl can be separated from those combinations by extractive distillation or sorption on activated carbon. A number of the combinations may boil too close together to separate by distillation, forming zeotropic blends (i.e., blends boiling within a limited temperature range). Some of the combinations may form binary or even ternary azeotropes. The azeotropes, zeotropes and individual compounds can be collected from different parts of a distillation column.

Without further elaboration, it is believed that one skilled in the art can, using the description herein, utilize the present invention to its fullest extent. The following specific embodiments are to be construed as illustrative, and not as constraining the remainder of the disclosure in any way whatsoever.

### EXAMPLES

### Hydrodehalogenation Catalyst Preparation for the Examples

Aqueous calcium nitrate (2.7 moles) is mixed with aqueous potassium fluoride (5.4 moles), heated and stirred briefly at 100°C to form a slurry of CaF₂. To this slurry is added copper nitrate (1 mole), nickel nitrate (1 mole) and chromium nitrate (1 mole) as solids. The slurry is stirred at 70 to 80°C until the salts, other than CaF₂, dissolve. This is followed by adding 0.1 mole of aqueous potassium hydroxide over 1 hour and boiling the mixture briefly. The slurry is cooled to 40 to 50°C and filtered. The solid is washed exhaustively to reduce the potassium content to an undetectable level. After drying, potassium hydroxide is added as a solution in quantities sufficient to provide a catalyst containing 9 weight % potassium. After drying again, the catalyst is calcined at 600°C for 8 to 16 hours, then granulated and screened to 1 to 2 mm particles. The catalyst is mixed with 1 to 5 wt% "Sterotex" powdered lubricant (registered trademark of Capital City Products Co., Columbus Ohio, division of Stokely-Van Camp, for its edible hydrogenated vegetable oil) to give 1/8" x 1/8" (3.2 mm x 3.2 mm) cylindrical pellets from a Stokes tablet machine.

### General Procedure for Product Analysis for the Examples

The products leaving the reactor were analyzed on line using a gas chromatograph. The column consisted of a 20'(6.1 m) x 1/8" (3.2 mm) stainless steel tube containing Krytox™ perfluorinated polyether on an inert support. Helium was used as the carrier gas. The product analyses are reported in mole%.

### Legend:

| | |
|---|---|
| 143a is CF₃CH₃ | 125 is CF₃CHF₂ |
| 216aa is CF₃CCl₂CF₃ | 226da is CF₃CHClCF₃ |
| 236fa is CF₃CH₂CF₃ | 235da is CF₃CHClCHF₂ |
| 225da is CF₃CHClCClF₂ | 245fa is CF₃CH₂CHF₂ |
| 254fb is CF₃CH₂CH₂F | 1225zc is CF₃CH=CF₂ |
| 1215xc is CF₃CCl=CF₂ | CT is contact time |
| HFP is CF₃CF=CF₂ | conv. is conversion |
| | sel. is selectivity |

### EXAMPLE 1

A 15" (381 mm) X 1/4" (6.4 mm) O.D. Inconel™ 600 nickel alloy U-tube reactor used for hydrodehalogenation (HDH) was charged with catalyst (21.7 g, 18 mL) pellets prepared substantially in accordance with the Catalyst Preparation described above. The HDH catalyst was in use for a variety of runs totaling 646 hours prior to its use in Example 1. Before starting the runs shown in Example 1, the HDH catalyst was regenerated with 50 sccm (8.3 x 10⁻⁷ m³/s) 50% air/50% N₂ at 350°C for 1 hour; 50 sccm (8.3 x 10⁻⁷ m³/s) air at 400°C for 2 hours; followed by purging with 200 sccm (3.3 x 10⁻⁶ m³/s) N₂ at 400°C for 40 minutes; and finally reduced with 50 sccm (8.3 x 10⁻⁷ m³/s) H₂ at 300°C for 75 minutes.

CF₃CCl₂CF₃ and H₂ were contacted with the catalyst at 350°C or 360°C and with a molar ratio of H₂:CF₃CCl₂CF₃ as shown in the table. Runs 1 to 3 were conducted at a pressure of 40 psig (380 kPa) and runs 4 to 9 were done at 100 psig (790 kPa). Results of the HDH reaction are shown in Table 1.

**TABLE 1**

| Run No. | HDH T(°C) | Molar Ratio H₂:216aa | CT Min. | % Conv. 216aa | %Sel. to | | | | |
|---|---|---|---|---|---|---|---|---|---|
| | | | | | 1225zc | 236fa | 245fa | 1215xc | 226da |
| 1 | 350 | 7 | 0.58 | 92.9 | 12.5 | 0.1 | 0.2 | 75.9 | 6.4 |
| 2 | 350 | 12 | 0.58 | 99.3 | 15.7 | 0.3 | 0.2 | 72.9 | 5.4 |
| 3 | 360 | 12 | 0.57 | 99.5 | 19.1 | 0.5 | 0.3 | 71.6 | 3.4 |
| 4 | 360 | 10 | 0.54 | 99.3 | 23.0 | 0.6 | 0.4 | 67.4 | 3.7 |
| 5 | 360 | 22 | 0.55 | 99.4 | 21.4 | 0.6 | 0.4 | 72.6 | 1.2 |
| 6 | 350 | 22 | 0.56 | 99.4 | 14.6 | 0.4 | 0.2 | 78.4 | 2.8 |
| 7 | 350 | 11 | 0.56 | 99.0 | 11.1 | 0.3 | 0.1 | 78.8 | 6.5 |
| 8 | 360 | 11 | 0.55 | 99.2 | 12.5 | 0.3 | 0.2 | 77.1 | 6.6 |
| 9 | 360 | 22 | 0.55 | 99.4 | 17.3 | 0.5 | 0.2 | 76.8 | 1.9 |

| | | | | | | | | | |
|---|---|---|---|---|---|---|---|---|---|
| ¹HDH is the hydrodehalogenation catalyst | | | | | | | | | |

### EXAMPLE 2

A second reactor for hydrogenation was added to the system on the exit side of the HDH reactor. This reactor consisted of an Inconel^{™} 600 tube 3/8" (9.5 mm) od. x 0.035" (0.9 mm) wall, 30" (762 mm) long packed with 21.8 grams (18.0 mL) of (0.5 weight% palladium on acid washed 12-30 mesh carbon (1.68-0.59 mm). The catalyst was treated with 50 sccm (8.3 x 10⁻⁷ m³/s) of hydrogen at 100°C for 15 hours prior to the run. The HDH reactor was the same as that used in Example 1. It was packed with 21.7 g of the HDH catalyst and treated with 50 sccm (8.3 x 10⁻⁷ m³/s) at 350°C for 15 hours prior to the run. The entire effluent of the HDH reactor (including HCl and HF) was passed into the second (hydrogenation) reactor. The contact time in the HDH reactor was 0.27 minutes except for runs 4 and 5 where it was 0.29 minutes. The temperature for the HDH reaction was 350°C for all the runs. The contact time (CT) shown in the table is for the Pd/C catalyst. The pressure of both reactors was 40 psig (377 kPa). Results of these reactions are shown in the Table 2.

**TABLE 2**

| Run No. | Pd/C T(°C) | Molar Ratio H₂:216aa | CT Min. | %Conv. 216aa | % Sel. to | | | | | |
|---|---|---|---|---|---|---|---|---|---|---|
| | | | | | 1225zc | 245fa | 254fb | 226da | 235da | 225da |
| 1 | 100 | 16 | 0.45 | 99.5 | 24.3 | 57.5 | <0.1 | 2.9 | 12.1 | 0.7 |
| 2 | 100 | 8 | 0.45 | 99.4 | 40.3 | 40.0 | 0.6 | 4.3 | 10.7 | 1.0 |
| 3 | 80 | 8 | 0.48 | 99.1 | 40.9 | 25.4 | 0.5 | 5.1 | 13.7 | 1.4 |
| 4 | 80 | 15 | 0.50 | 99.2 | 42.7 | 22.7 | 0.4 | 4.3 | 15.2 | 1.2 |
| 5 | 150 | 15 | 0.42 | 99.5 | 1.5 | 85.6 | 1.2 | 3.3 | 5.5 | 1.1 |
| 6 | 150 | 16 | 0.40 | 99.6 | 0.4 | 88.7 | 1.2 | 3.2 | 4.0 | 1.2 |
| 7 | 150 | 8 | 0.40 | 99.5 | 5.7 | 80.7 | 1.0 | 5.2 | 3.7 | 2.2 |
| 8 | 140 | 16 | 0.41 | 99.5 | 1.8 | 86.3 | 1.0 | 3.4 | 4.3 | 2.0 |
| 9 | 145 | 16 | 0.40 | 99.5 | 1.5 | 86.1 | 0.9 | 3.4 | 4.9 | 2.0 |
| 10 | 150 | 16 | 0.40 | 99.5 | 2.5 | 86.1 | 1.0 | 3.3 | 4.4 | 1.6 |
| 11 | 150 | 16 | 0.40 | 99.5 | 0.6 | 88.5 | 1.1 | 2.3 | 4.7 | 1.4 |

### EXAMPLE 3

A second reactor for hydrogenation was added to the system on the exit side of the HDH reactor. This reactor consisted of an Inconel^{™} 600 tube 3/8" (9.5 mm) od. x 0.035" (0.9 mm) wall, 30" (762 mm) long packed with 27.8 grams (35.9 mL) of (0.5 weight% palladium on acid washed 12-30 mesh carbon (1.68-0.59 mm). The catalyst was treated with 120 sccm (2.0 x 10⁻⁶ m³/s) of hydrogen at 150°C for one hour prior to the run. The HDH reactor was the same as that used in Example 1. It was packed with 21.7 g of the HDH catalyst and treated with 120 sccm (2.0 x 10⁻⁶ m³/s) of hydrogen at 350°C for one hour prior to the run. The entire effluent of the HDH reactor (including HCl and HF) was passed into the second (hydrogenation) reactor. The contact time in the HDH reactor was 0.14 minutes for run 1, 0.13 minutes for runs 2, 3 and 6, and 0.07 minutes for runs 4 and 5. The temperature for the HDH reaction was 350°C for run 1, 355°C for runs 2, 3, 4 and 6 and 360°C for run 5. The contact time (CT) shown in the table is for the Pd/C catalyst. The temperature of the hydrogenolysis reaction was 150°C for all runs. The pressure of both reactors was 40 psig (377 kPa). Results of these reactions are shown in the Table 3.

**TABLE 3**

| Run No. | Molar Ratio H₂:216aa | CT Min. | %Conv. 216aa | %Sel. to | | | | | | |
|---|---|---|---|---|---|---|---|---|---|---|
| | | | | 1225zc | 236fa | 245fa | 254fb | 226da | 235da | 225da |
| 1 | 16 | 0.25 | 100 | 0.6 | 6.1 | 87.9 | 1.6 | 1.3 | 0.8 | 0.2 |
| 2 | 16 | 0.25 | 100 | 0.5 | 5.9 | 88.1 | 1.6 | 1.5 | 1.0 | <0.1 |
| 3 | 16 | 0.25 | 100 | 0.4 | 6.5 | 86.0 | 1.7 | 2.1 | 1.8 | <0.1 |
| 4 | 16 | 0.13 | 100 | 0.5 | 19.7 | 68.9 | 1.4 | 6.4 | 1.6 | <0.1 |
| 5 | 16 | 0.13 | 100 | 0.4 | 18.0 | 69.1 | 1.4 | 7.5 | 2.0 | <0.1 |
| 11 | 16 | 0.25 | 100 | 0.3 | 10.2 | 77.7 | 1.7 | 5.4 | 3.2 | <0.1 |

### EXAMPLE 4

The reactor used for hydrodehalogenation (HDH) in Example 1 was charged with catalyst (24.1 g, 18.0 mL) 1/8" x 1/8" (3.2 mm x 3.2 mm) pellets prepared substantially in accordance with the Catalyst Preparation above. The HDH catalyst unlike that of Example 1 was not used previously, i.e., it was a fresh catalyst. Before starting the runs shown in Example 1, the HDH catalyst was treated with 110 sccm (1.8 x 10⁻⁶ m³/s) H₂ at 350°C and 40 psig (380 kPa) for one hour.

CF₃CCl₂CF₃ and H₂ were contacted with the catalyst at 350°C or 360°C and with a molar ratio of H₂:CF₃CCl₂CF₃ as shown in the table. All runs were conducted at a pressure of 40 psig (380 kPa). Results of the HDH reaction are shown in Table 4.

**TABLE 4**

| Run No. | HDH¹ T(°C) | Molar Ratio H₂:216aa | CT Min. | %Conv. 216aa | 1225zc | %Sel. to 1215xc | Other² |
|---|---|---|---|---|---|---|---|
| 1 | 350 | 16 | 0.27 | 100 | 9.1 | 87.4 | 3.6 |
| 2 | 350 | 16 | 0.27 | 100 | 10.2 | 86.2 | 3.7 |
| 3 | 350 | 8 | 0.27 | 100 | 12.0 | 84.0 | 4.0 |
| 4 | 350 | 16 | 0.27 | 100 | 10.9 | 84.8 | 4.2 |

| | | | | | | | |
|---|---|---|---|---|---|---|---|
| ¹HDH is the hydrodehalogenation catalyst ²Other includes 236fa, 245fa and 226da | | | | | | | |

### EXAMPLE 5

The reactor and catalyst used for hydrodehalogenation (HDH) in Example 3 was used. The HDH catalyst was treated prior to use with hydrogen at 300°C for one hour; 67% air/33% N₂ at 300°C for 1 hour; 50% air/50% N₂ at 400°C for 1 hour; air at 450°C for 2 hours; air at 500°C for 5 hours; air at 550°C for 5 hours; followed by purging with N₂ at 350°C for 3 hours; N₂/H₂ (1:1) at 350°C for one hour; and finally reduced with H₂ at 350°C for one hour.

CF₃CCl₂CF₃ and H₂ were contacted with the catalyst at 350°C and with a molar ratio of H₂:CF₃CCl₂CF₃ as shown in the Table 4. The HDH reaction was done at a pressure of 40 psig (380 kPa) and a contact time of 0.27 minutes. The entire effluent (including HCl and HF) was then passed into a second (hydrogenation) reactor which was the same as that used in Example 3 and contained the same catalyst. The hydrogenation catalyst was purged with hydrogen at 380 kPa and 150°C, the same temperature and pressure as the hydrogenation reaction, for 8 hours prior to use. The contact time of the hydrogenation reaction was 0.51 minutes. The results of the combined reactions are shown in Table 5.

**TABLE 5**

| Run No. | Molar Ratio H₂:216aa | %Conv. 216aa | %Sel. to | | | | | |
|---|---|---|---|---|---|---|---|---|
| | | | 1225zc | 245fa | 254fb | 226da | 235da | Other¹ |
| 1 | 16 | 100 | 0.0 | 89.0 | 2.7 | 0.1 | 4.6 | 3.6 |
| 2 | 8 | 100 | 3.8 | 80.6 | 2.1 | 1.0 | 9.2 | 3.3 |
| 3 | 8 | 100 | 4.8 | 81.3 | 2.0 | 1.2 | 7.6 | 2.9 |

| | | | | | | | | |
|---|---|---|---|---|---|---|---|---|
| ¹Other includes 236fa and 1225xc | | | | | | | | |

### EXAMPLE 6

The reactor and catalyst used for hydrodehalogenation (HDH) in Example 4 was used. The HDH catalyst was treated prior to use in the same way as the catalysts used in Example 4.

CF₃CCl₂CF₃ and H₂ were contacted with the catalyst at 350°C and with a molar ratio of H₂:CF₃CCl₂CF₃ as shown in the Table 4. The HDH reaction was done at a pressure of 40 psig (380 kPa) and a contact time of 0.27 minutes except for run 3 which was done at 0.13 minutes. The entire effluent (including HCl and HF) was then passed into a second (hydrogenation) reactor which was the same as that used in Example 3 and contained the same catalyst. The hydrogenation catalyst was purged with hydrogen at 380 kPa and 150°C, the same temperature and pressure as the hydrogenation reaction, for 8 hours prior to use. The contact time of the hydrogenation reaction was 0.51 minutes except for run 3 which was 0.25 minutes. The results of the combined reactions are shown in Table 6.

**TABLE 6**

| Run No. | Molar Ratio H₂:216aa | %Conv. 216aa | %Sel. to | | | | | |
|---|---|---|---|---|---|---|---|---|
| | | | 1225zc | 245fa | 254fb | 226da | 235da | Other¹ |
| 1 | 16 | 100 | 0.4 | 81.0 | 3.7 | 1.0 | 9.4 | 0.2 |
| 2 | 16 | 100 | 1.1 | 73.9 | 3.7 | 1.0 | 14.0 | 0.2 |
| 3 | 16 | 100 | 7.4 | 74.1 | 2.5 | 1.0 | 11.6 | 0.2 |
| 4 | 8 | 100 | 11.5 | 66.4 | 1.8 | 1.8 | 10.1 | 0.2 |

| | | | | | | | | |
|---|---|---|---|---|---|---|---|---|
| ¹Other includes 236fa and 1225xc | | | | | | | | |

### EXAMPLE 7

### CF₃CH=CF₂ + HF → CF₃CH₂CF₃ (Liquid Phase)

A Hastelloy™ C nickel alloy (160 mL) autoclave equipped with a magnetically driven stirrer, pressure transducer, thermocouple well, and vapor inlet valve was evacuated, cooled in liquid nitrogen, and charged with anhydrous HF (50 g, 2.5 moles). 1,1,3,3,3-Pentafluoro-1-propene (20.2 g, 0.15 mole) was added to the autoclave at a temperature of about -31 °C from a small cylinder. The autoclave was then warmed to ambient temperature. The contents of the autoclave were stirred at about 500 rpm and the autoclave was heated to 70°C over the course of about 8 minutes. The pressure rose to 106 psig (832 kPa) and then leveled out at 99 psig (784 kPa). After 2 hours at 71°C and 99 psig (784 kPa), a sample of the vapor phase of the autoclave was analyzed by GC-MS (see Table 7).

**TABLE 7**

| Component | GC Area % |
|---|---|
| C₃F₆ | 0.3 |
| 1225zc | 84.9 |
| 236fa | 14.3 |

### EXAMPLE 8

### CF₃CH=CF₂ + HF → CF₃CH₂CF₃ (Vapor Phase)

A 15 mL tubular reactor was packed with 13.7 g of a catalyst comprising a 0.02:0.98:1.0 ratio of CoF₂, ZnF₂, and AlF₃, respectively. The catalyst was activated by heating to 250°C over the course of 1 hour while passing 50 sccm (8.3 x 10⁻⁷ m³/s) nitrogen through the reactor. The nitrogen flow was then reduced to 20 sccm (3.3 x 10⁻⁷ m³/s) and HF was admitted at 50 sccm (8.3 x 10⁻⁷ m³/s) at 250°C for 1 hour.

A 4:1 molar mixture of HF and 1,1,3,3,3-pentafluoro-1-propene was fed to the reactor with a catalyst contact time of 15 seconds at 300°C. A comparison of the reactor feed gas and effluent under these conditions is given in Table 8.

**TABLE 8**

| Temp. °C | 143a | HFP | 1225zc | 236fa | 125 |
|---|---|---|---|---|---|
| Feed | 0.0 | 0.2 | 98.4 | 0.6 | 0.0 |
| 350 | 0.2 | 0.1 | 1.4 | 97.8 | 0.1 |
| 300 | 0.1 | 0.1 | 0.4 | 98.9 | 0.0 |

Other products observed by GC include CH₃Cl, CH₃F, CHF₂CClF₂, and CHClFCF₃ which appear to be contaminants in the 1225zc.

## Claims

1. A process for producing compositions comprising (c1) a compound selected from the group consisting of CF₃CHFCF₃, CF₃CH₂CF₃ and CHF₂CH₂CF₃ and (c2) at least one saturated compound selected from halogenated hydrocarbons and ethers having the formula
CₙH_{2n+2-a-b}ClₐF_{b}O_{c}
wherein n is an integer from 1 to 4, a is an integer from 0 to 2n+1, b is an integer from 1 to 2n+2-a, and c is 0 or 1, provided that when c is 1 then n is an integer from 2 to 4, and provided that component (c2) does not include the selected component (c1) compound wherein the molar ratio of component (c2) to component (c1) is between about 1:99 and a molar ratio of HF to component (c1) in an azeotrope or azeotrope-like composition of component (c1) with HF, comprising:
(A) combining (i) said azeotrope or azeotrope-like composition with (ii) at least one fluorination precursor compound wherein the precursor component (ii) is the fluorination precursor to component (c2); and
(B) reacting a sufficient amount of the HF from the azeotrope or azeotrope-like composition (i) with precursor component (ii) to provide a composition containing components (c1) and (c2) in said ratio.

2. The process of Claim 1 wherein component (c1) is CF₃CHFCF₃; wherein the molar ratio of component (c2) to CF₃CHFCF₃ is between about 1:99 and about 41.3:58.7; and wherein in (A) an azeotrope or azeotrope-like composition consisting essentially of CF₃CHFCF₃ and HF wherein the ratio of HF to CF₃CHFCF₃ is at least about the desired ratio is combined with (ii).

3. The process of Claim 1 wherein component (c1) is CF₃CH₂CF₃; wherein the molar ratio of component (c2) to CF₃CH₂CF₃ is between about 1:99 and about 59:41; and wherein in (A) an azeotrope or azeotrope-like composition consisting essentially of CF₃CH₂CF₃ and HF wherein the ratio of HF to CF₃CH₂CF₃ is at least about the desired ratio is combined with (ii).

4. The process of Claim 1 wherein component (c1) is CHF₂CH₂CF₃; wherein the molar ratio of component (c2) to CHF₂CH₂CF₃ is between about 1:99 and about 84:16; and wherein in (A) an azeotrope or azeotrope-like composition consisting essentially of CHF₂CH₂CF₃ and HF wherein the ratio of HF to CHF₂CH₂CF₃ is at least about the desired ratio is combined with (ii).

5. A composition comprising:
(c1) a compound selected from the group consisting of CF₃CHFCF₃, CF₃CH₂CF₃ and CHF₂CH₂CF₃; and
(c2) at least two saturated compounds selected from halogenated hydrocarbons and ethers having the formula:
CₙH_{2n+2-a-b}ClₐF_{b}O_{c}
wherein n is an integer from 1 to 4, a is an integer from 0 to 2n+1, b is an integer from 1 to 2n+2-a, and c is 0 or 1, provided that when c is 1 then n is an integer from 2 to 4, and provided that component (c2) does not include the selected component (c1) compound and provided that c is 1 for at least one of the component (c2) compounds, wherein the molar ratio of component (c2) to component (c1) is between 1:99 and 41.3:58.7 when component (c1) is CF₃CHFCF₃, between 1:99 and 59:41 when component (c1) is CF₃CH₂CF₃, and between 1:99 and 84:16 when component (c1) is CHF₂CH₂CF₃.
